Europäisches Patentamt

European Patent Office

Office européen des brevets

(19)

(11) Veröffentlichungsnummer: **0 097 243**
**B1**

(12) **EUROPÄISCHE PATENTSCHRIFT**

(45) Veröffentlichungstag der Patentschrift:
10.07.85

(21) Anmeldenummer: **83104790.7**

(22) Anmeldetag: **16.05.83**

(51) Int. Cl.⁴: **C 07 C 118/00, C 07 C 119/048**

(54) **Verfahren zur Abtrennung von technisch reinem 2,4-Diisocyanatotoluol oder von, einen erhöhten Gehalt an 2,4-Diisocyanatotoluol aufweisenden, Isomerengemischen von, im wesentlichen aus 2,4- und 2,6-Diisocyanatotoluol bestehenden, Isomerengemischen.**

(30) Priorität: **29.05.82 DE 3220439**

(43) Veröffentlichungstag der Anmeldung:
**04.01.84 Patentblatt 84/1**

(45) Bekanntmachung des Hinweises auf die Patenterteilung:
**10.07.85 Patentblatt 85/28**

(84) Benannte Vertragsstaaten:
**BE DE FR GB IT NL**

(56) Entgegenhaltungen:
**EP - A - 0 023 015**
**US - A - 3 217 024**

(73) Patentinhaber: **BAYER AG, Konzernverwaltung RP Patentabteilung, D-5090 Leverkusen 1 Bayerwerk (DE)**

(72) Erfinder: **Mitrowsky, Alexander, Dr., Moselstrasse 19, D-4047 Dormagen (DE)**
Erfinder: **Wissner, Adolf, Dr., Am Wasserturm 13, D-5090 Leverkusen 3 (DE)**
Erfinder: **Hauser, Werner, Dr., Quellenweg 8, D-5068 Odenthal (DE)**

**Beschreibung**

Die Erfindung betrifft ein verbessertes, einstufiges Verfahren zur Isolierung von technisch reinem 2,4-Diisocyanatotoluol bzw. von Isomerengemischen mit einem erhöhten Gehalt an 2, 4-Diisocyanatotoluol aus, im wesentlichen aus 2,4- und 2,6-Diisocyanatotoluol bestehenden Isomerengemischen durch einstufige Kristallisation in einem Röhrenkristaller.

Bei der großtechnischen Herstellung von Diisocyanatotoluol, einem wichtigen Ausgangsmaterial für die Herstellung von Polyurethankunststoffen, fallen im allgemeinen Gemische der 2,4- und 2,6-Isomeren an. Da die beiden Isomeren sich jedoch bei der Weiterverarbeitung chemisch nicht gleich verhalten, entstehen je nach Isomerenzusammensetzung der Isomerengemische Endprodukte unterschiedlicher Eigenschaften. Oft ist es auch zweckmäßig, bei der Herstellung von Polyurethankunststoffen technisch reines, d. h. einen Reinheitsgrad von mindestens 98 Gew.-% aufweisendes, 2,4-Diisocyanatotoluol einzusetzen.

Da die Einstellung des jeweils erwünschten Isomerenverhältnisses oder die Reindarstellung auf dem direkten Produktionsweg aufwendig und teuer ist, mangelte es nicht an Versuchen, dieses Ziel durch technisch gangbare Methoden der Isomerentrennung, insbesondere auf dem Weg der Kristallisation, zu erreichen.

So wird beispielsweise in US-PS 3 217 024 ein zweistufiges Kristallisationsverfahren beschrieben, bei welchem aus einem 80/20-Isomerengemisch in der ersten Stufe ein 90—94%iges 2,4-Diisocyanatotoluol und ein 65/35-Isomerengemisch gewonnen wird, wonach das noch unreine 2,4-Isomere in einer 2. Stufe auf die geforderte Reinheit gebracht wird. Der Grund, weswegen die Autoren dieser Veröffentlichung diesen umständlichen Weg beschritten, ist vermutlich in der starken Neigung von 2,4-Diisocyanatotoluol, aus Isomerengemischen erst nach starker Unterkühlung und unter Bildung von Einschlüssen zu kristallisieren, zu sehen.

Die Autoren der US-PS 4 246 187 versuchten diese Schwierigkeiten dadurch zu umgehen, daß sie jeweils nur 10—20 Gew.-% des 2,4-Diisocyanatotoluols auskristallisieren ließen und diesen Anteil dann mit Hilfe einer Zentrifuge abtrennten. Es versteht sich, daß auch dieses Verfahren zur Isomerentrennung bzw. -anreicherung mit einem erheblichen technischen Aufwand verbunden ist.

Auch eine Übertragung des in »Chemische Technologie«, 3, Carl Hanser Verlag/München, (1959) auf Seiten 812—13 insbesondere zur Isomerentrennung von Kernchlorverbindungen beschriebenen »Abtropfverfahrens« auf die Trennung von Diisocyanatotoluol-Isomeren führt zu keinem befriedigendem Ergebnis, da ein Abkühlen bis an den Erweichungspunkt des Eutektikums eine nur unbefriedigende Isomerentrennung bei zudem schlechter Raum/Zeit-Ausbeute zur Folge hat.

Überraschenderweise wurde nunmehr gefunden, daß mit dem nachstehend näher beschriebenen erfindungsgemäßen Verfahren eine einfache, einstufig durchführbare Abtrennung von technisch reinem 2,4-Diisocyanatotoluol bzw. von Isomerengemischen mit einem erhöhten Gehalt an 2,4-Diisocyanatotoluol von, im wesentlichen aus 2,4- und 2,6-Diisocyanatotoluol bestehenden Isomerengemischen möglich ist.

Gegenstand der Erfindung ist ein Verfahren zur Abtrennung von reinem 2,4-Diisocyanatotoluol oder von, einen erhöhten Gehalt an 2,4-Diisocyanatotoluol aufweisenden, Isomerengemischen von, im wesentlichen aus 2,4- und 2,6-Diisocyanatotoluol bestehenden Isomerengemischen, mit einem Gehalt an 2, 4-Diisocyanatotoluol von 65—85 Gew.-% und an 2,6-Diisocyanatotoluol von 15—35 Gew.-%, dadurch gekennzeichnet, daß man das als Ausgangsmaterial einzusetzende Isomerengemisch in einem Röhrenkristaller, bestehend aus einem oder mehreren aufrechtstehenden, parallel geschalteten Rohren, an denen bzw. zwischen denen ein gleichmäßiger und ungehinderter Kühl- bzw. Heizmittelzutritt möglich ist,

(i)     auf eine Temperatur von +4°C bis +9°C abkühlt, anschließend

(ii)    das Gemisch unter Einhaltung eines Temperaturgradienten von 0,5—3°C/h auf —2 bis —6°C abkühlt und anschließend mindestens 30 Minuten in diesem Temperaturbereich beläßt, anschließend

(iii)   die vorliegende Mutterlauge bei dieser Temperatur ablaufen läßt und

(iv)    die danach im Röhrenkristaller vorliegende Festsubstanz unter Erwärmen zum Aufschmelzen bringt.

Die nach diesem Verfahren im Verfahrensschritt (iii) anfallende Festsubstanz weist bereits, im Vergleich zum Ausgangsgemisch, einen wesentlich erhöhten Gehalt an 2,4-Diisocyanatotoluol auf. So wäre es beispielsweise möglich, allein durch die während der Verfahrensschritte (i) und (ii) stattfindende selektive Kristallisation aus einem, im wesentlichen aus 65 Gew.-% 2,4- und 35 Gew.-% 2,6-Diisocyanatotoluol bestehenden, Isomerengemisch ein aus ca. 80 Gew.-% 2,4- und ca. 20 Gew.-% 2,6-Diisocyanatotoluol bestehendes Isomerengemisch als Festsubstanz zu erhalten und nach Aufschmelzen aus dem Röhrenkristaller zu isolieren.

Eine noch weitergehende Isomerentrennung kann auf einfache Weise dadurch erzielt werden, daß man die nach dem Verfahrensschritt (iii) im Röhrenkristaller vorliegende Festsubstanz allmählich unter Einhaltung eines Temperaturgradienten von 0,5—3°C/h unter fortlaufendem Ablaufen von hierbei gebildeter Flüssigkeit partiell aufschmilzt bis der noch im Röhrenkristaller verbleibende Festkörper dem gewünschten Gehalt an 2,4-Diisocyanatotoluol aufweist. Auf diese Weise ist es beispielsweise möglich, von einem Isomerengemisch mit einem Gehalt an 2,4-Diisocyanatotoluol von 75 bis 85, vorzugsweise 78 bis 82 Gew.-% und einem Gehalt an 2,6-Diisocyanatotoluol von 15 bis 25, vorzugsweise 18 bis 22 Gew.-% ausgehend, technisch reines, d. h. einen Reinheitsgrad von mindestens 98 Gew.-%, vorzugsweise mindestens 99 Gew.-%, aufweisendes 2,4-Diisocyanatotoluol, als noch im Röhrenkristaller verbleibende Festsubstanz zu erhalten und nach Aufschmelzen als Flüssigkeit zu isolieren.

Bei der Durchführung des erfindungsgemäßen Verfahrens werden als Ausgangsmaterial technische Diisocyanatotoluol-Isomerengemische mit der bereits obengenannten Zusammensetzung eingesetzt. Vorzugsweise weisen die als Ausgangsmaterial eingesetzten Gemische einen Gehalt an 2,4-Diisocyanatotoluol von 75 bis 85, insbesondere 78 bis 82 Gew.-% und einen Gehalt an 2,6-Diisocyanatotoluol von 15 bis 25, vorzugsweise 18 bis 22 Gew.-% auf.

Bei der Durchführung des erfindungsgemäßen Verfahrens kann das Ausgangsgemisch im Röhrenkristaller ohne besondere Vorsichtsmaßnahmen (beispielsweise Einhaltung eines bestimmten Temperaturgradienten) rasch bis zum Beginn der Kristallisation, d. h. auf eine Temperatur von +4°C bis +9°C, vorzugsweise +5°C bis +7°C, abgekühlt werden. Nach Erreichen dieser Temperatur kühlt man unter Einhaltung eines Temperaturgradienten von 0,5—3°C/h das Gemisch bis auf eine Temperatur von ca. −2°C bis ca. −6°C, vorzugsweise −3°C bis −5°C ab. Hierbei ist es wesentlich, daß der Schmelzpunkt des Eutektikums (−7°C) nicht erreicht wird, da nur unter dieser Voraussetzung eine optimale Isomerentrennung möglich ist. In einem nächsten Verfahrensschritt wird dann durch eine Verweilzeit von mindestens 30 Minuten, vorzugsweise von 1 bis 3 Stunden, innerhalb des letztgenannten Temperaturbereichs ein praktisch vollständiger Temperaturausgleich in dem Röhrenkristaller herbeigeführt. Während dieser Zeit wird die Temperatur kühlmittelseitig konstant gehalten. Nach der Verweilzeit läßt man die Mutterlauge ablaufen, die man beispielsweise in einem Kessel mit Umpumpvorrichtung auffängt.

Gemäß einer Ausführungsform des erfindungsgemäßen Verfahrens wird, wie oben bereits ausgeführt, der dann noch im Kristaller vorliegende Feststoff durch Aufschmelzen isoliert. Gemäß der bevorzugten Ausführungsform des erfindungsgemäßen Verfahrens zur Herstellung von technisch reinem 2,4-Diisocyanatotoluol wird jedoch die nach Ablauf der Mutterlauge im Röhrenkristaller befindliche Festsubstanz allmählich unter Einhaltung eines Temperaturgradienten von 0,5—3°C/h unter fortlaufendem Ablauf von gebildeter Flüssigkeit erwärmt, bis der noch im Röhrenkristaller verbleibende Feststoff den gewünschten Gehalt an 2,4-Diisocyanatotoluol aufweist. Bei der Verwendung des besonders bevorzugten Ausgangsgemischs mit einem Gehalt an 2,4-Diisocyanatotoluol von 78 bis 82 Gew. %, kann davon ausgegangen werden, daß die noch im Röhrenkristaller verbleibende Festsubstanz technisch reines 2,4-Diisocyanatotoluol darstellt, wenn die vereinigten Mutterlaugen und Schmelzen einen Gehalt an 2,4-Diisocyanatotoluol von mindestens 63, vorzugsweise 63 bis 68 und insbesondere von 64 bis 66 Gew.-% aufweisen. Falls hiernach noch weiterer Ablauf anfällt, kann dieser separat aufgefangen und bei der nächsten Charge mit eingesetzt werden. Im allgemeinen kann zur Abtrennung vom technisch reinen 2,4-Diisocyanatotoluol der allmähliche Aufschmelzvorgang bei dem letztgenannten Gehalt der vereinigten Mutterlauge und Schmelzen an 2,4-Diisocyanatotoluol abgebrochen werden. Der Ablauf wird geschlossen und die noch im Kristaller befindliche Substanz aufgeschmolzen und in einen Lagertank für technisch reines 2,4-Diisocyanatotoluol abgelassen. Wie leicht ersichtlich, kann die gewünschte Reinheit durch früheres oder späteres Schließen des Ablaufs beeinflußt werden.

Wie bereits dargelegt, besteht der beim erfindungsgemäßen Verfahren verwendete Röhrenkristaller im wesentlichen aus einem oder vorzugsweise mehreren aufrechtstehenden, vorzugsweise senkrecht angeordneten, parallel geschalteten Rohren, an denen bzw. zwischen denen ein gleichmäßiger und ungehinderter Kühl- bzw. Heizmittelzutritt möglich ist. Im allgemeinen weisen die einzelnen Rohre eine Länge von 1 bis 8, vorzugsweise 3 bis 6 m und einen lichten Durchmesser von 1 bis 8, vorzugsweise 2 bis 5 cm auf. Die Anzahl der Rohre und deren Dimension ist jedoch keineswegs erfindungswesentlich und hängt vor allem von der gewünschten Kapazität der Apparatur ab. So kann z. B. als Laborapparatur auch ein einzelnes Rohr mit einer Länge von nur 50 cm verwendet werden. Als Heiz- und Kühlmedium kann sowohl eine geeignete Flüssigkeit wie auch Luft oder ein anderes Gas verwendet werden. Sowohl Flüssigkeit als auch Gas werden im Zwangsumlauf geführt. Zur Kühlung des Wärmeübertragungsmediums können flüssiges Ammoniak oder auch Frigen verwendet werden, wobei der Wärmeaustausch über ein entsprechendes Kühlregister erfolgt. Als Heizmittel kann ein organischer Wärmeträger oder Wasserdampf eingesetzt werden, wobei der Wärmeaustausch über ein entsprechendes Heizregister erfolgt. Selbstverständlich muß für die Energieübertragung ein geeignetes Feinreguliersystem vorhanden sein, um die in der Kühl- und Heizphase erforderlichen kleinen Temperaturgradienten erreichen zu können.

Die in den nachfolgenden Beispielen genannten Prozentangaben beziehen sich auf Gewichtsprozente.

Beispiel 1

Der verwendete Röhrenkristaller mit einem Volumen von 20 m$^3$ bestand im wesentlichen aus einem ummantelten Rohrbündel aus 1700 parallel angeordneten, senkrecht stehenden Rohren einer Länge von 6 m und eines lichten Durchmessers von 5 cm. Das Rohrbündel war oben mit einem gemeinsamen Zulauf und unten mit einem gemeinsamen Ablauf verbunden. Der Röhrenkristaller war zudem mit 3 Thermoelementen ausgerüstet, die in verschiedener Höhe der Rohre angebracht waren. Das Wärmeübertragungsmedium war Luft, die Kühlung erfolgte mittels flüssigen Ammoniaks, die Heizung mittels Wasserdampf.

In diesem Kristaller wurden 20 m³ eines Gemischs aus 80,5% 2,4-Diisocyanatotoluol und 19,5% 2,6-Diisocyanatotoluol bei etwa 30°C eingefüllt. Innerhalb von 5 Stunden wird das Gemisch dann auf eine Temperatur von 6°C abgekühlt. Bei dieser Temperatur setzte eine partielle Kristallisation ein. Anschließend wird das Gemisch unter Einhaltung eines Temperaturgradienten von 1°C/h auf −5°C abgekühlt und während einer Stunde bei dieser Temperatur gehalten. Anschließend wird der Ablauf geöffnet und die Mutterlauge während weiterer 4stündiger Aufrechterhaltung der Temperatur von −5°C ablaufen gelassen. Anschließend wird die im Röhrenkristaller verbleibende Festsubstanz unter fortlaufendem Ablauf der gebildeten Schmelze unter Einhaltung eines Temperaturgradienten von 1°C/h auf 21°C erwärmt. Das Gemisch der Mutterlauge mit dem hierbei gebildeten Ablauf wies einen Gehalt an 2,4-Diisocyanatotoluol von 68,7% und einen Gehalt an 2,6-Diisocyanatotoluol von 31,3% auf. Nach Schließen des Ablaufs wird die noch im Kristaller befindliche Festsubstanz aufgeschmolzen und in einen Lagertank für technisch reines 2,4-Diisocyanatotoluol geleitet. Das Produkt wies einen Gehalt an 2,4-Diisocyanatotoluol von 99,2% auf.

### Beispiel 2

In einem Versuchskristaller mit einer Kapazität von 7 l, dessen wesentlicher Teil aus einem Rohr mit einer Länge von 3,7 m und eines lichten Durchmessers von 5 cm bestand, wurde ein Gemisch von 79,2% 2,4-Diisocyanatotoluol mit 80,8% 2,6-Diisocyanatotoluol eingefüllt. Innerhalb einer Zeitspanne von 3 Stunden wurde das Gemisch von der Einsatztemperatur von 25°C auf +6°C abgekühlt. Anschließend wurde unter Einhaltung eines Temperaturgradienten von 1°C/h das Gemisch auf −5°C abgekühlt. Dann wurde die Temperatur von −5°C während eines Zeitraums von 2 Stunden aufrechterhalten. Während eines weiteren Zeitraums von 3 Stunden bei −5°C erfolgte dann der Ablauf der Mutterlauge. Schließlich wurde die noch im Kristaller verbliebene Festsubstanz unter Einhaltung eines Temperaturgradienten von 1°C/h unter fortlaufendem Ablauf von hierbei gebildeter Schmelze auf 21°C erwärmt. Das Gemisch aus Mutterlauge und Schmelze wies danach einen Gehalt an 2,4-Diisocyanatotoluol von 65,3% auf. Das letztlich erhaltene technisch reine 2,4-Diisocyanatotoluol hatte einen Reinheitsgrad von 99,5%.

In diesem Beispiel wurde als Wärmeübertrager Methanol benutzt, das von einem Kryostaten gekühlt und von einem Thermostaten erwärmt wurde.

### Beispiel 3

In den in Beispiel 1 beschriebenen Röhrenkristaller wurden 20 m³ eines Isomerengemisches aus 65% 2,4- und 35% 2,6-Diisocyanatotoluol eingefüllt. Dann wurde während eines Zeitraums von 3 Stunden von der Einsatztemperatur von +25°C auf +6°C (beginnende Kristallisation) abgekühlt. Anschließend wird das Gemisch unter Einhaltung eines Temperaturgradienten von 1°C/h auf −4°C abgekühlt und dann während einer Verweilzeit von 2 Stunden bei dieser Temperatur belassen. Anschließend wurde die Mutterlauge unter weiterer Aufrechterhaltung der Temperatur von −4°C während eines Zeitraums von 5 Stunden ablaufen gelassen.

Die dann im Kristaller vorliegende Festsubstanz wurde aufgeschmolzen und in einen Lagertank geleitet. Sie bestand zu 79,2% aus 2,4-Diisocyanatotoluol und zu 20,8% aus 2,6-Diisocyanatotoluol.

### Patentansprüche

1. Verfahren zur Abtrennung von reinem 2,4-Diisocyanatotoluol oder von, einen erhöhten Gehalt an 2,4-Diisocyanatotoluol aufweisenden, Isomerengemischen von, im wesentlichen aus 2,4- und 2,6-Diisocyanatotoluol bestehenden Isomerengemischen mit einem Gehalt an 2,4-Diisocyanatotoluol von 65—85 Gew.-% und an 2,6-Diisocyanatotoluol von 15—35 Gew.-%, dadurch gekennzeichnet, daß man das als Ausgangsmaterial einzusetzende Isomerengemisch in einem Röhrenkristaller, bestehend aus einem oder mehreren aufrechtstehenden, parallel geschalteten Rohren, an denen bzw. zwischen denen ein gleichmäßiger und ungehinderter Kühl- bzw. Heizmittelzutritt möglich ist,

(i)  auf eine Temperatur von +4°C bis +9°C abkühlt, anschließend

(ii)  das Gemisch unter Einhaltung eines Temperaturgradienten von 0,5—3°C/h auf −2 bis −6°C abkühlt und anschließend mindestens 30 Minuten in diesem Temperaturbereich beläßt, anschließend

(iii)  die vorliegende Mutterlauge bei dieser Temperatur ablaufen läßt und

(iv)  die danach im Röhrenkristaller vorliegende Festsubstanz unter Erwärmen zum Aufschmelzen bringt.

2. Verfahren gemäß Anspruch 1 zur Herstellung von technisch reinem 2,4-Diisocyanatotoluol, dadurch gekennzeichnet, daß man

(iv)  die gemäß Schritt (iii) erhaltene, im

Röhrenkristaller vorliegende Festsubstanz durch Erwärmen unter Einhaltung eines Temperaturgradienten von 0,5—3°C/h teilweise zum Schmelzen bringt, bis die verbleibende Festsubstanz einen Gehalt an 2,4-Diisocyanatotoluol von mindestens 98% aufweist und anschließend

(v)   das so erhaltene, technisch reine 2,4-Diisocyanatotoluol nach Aufschmelzen aus dem Röhrenkristaller isoliert.

3. Verfahren gemäß Ansprüchen 1 und 2, dadurch gekennzeichnet, daß man als Ausgangsgemisch ein im wesentlichen aus 75 bis 85 Gew.-% 2,4-Diisocyanatotoluol und 15 bis 25 Gew.-% 2,6-Diisocyanatotoluol bestehendes Isomerengemisch verwendet.

## Claims

1. Process for separating pure 2,4-diisocyanatotoluene or isomer mixtures having a relatively high content of 2,4-diisocyanatotoluene from isomer mixtures consisting essentially of 2,4- and 2,6-diisocyanatotoluene and having a content of 2,4-diisocyanatotoluene of 65—85% by weight and of 2,6-diisocyanatotoluene of 15—35% by weight, characterised in that, in a tube crystalliser consisting of one or more vertical tubes arranged parallel to one another on which or between which it is possible for heating or cooling media to move uniformly and freely, the isomer mixture to be used as the starting material

(i)    is cooled to a temperature of +4°C to +9°C, then

(ii)   the mixture, while maintaining a temperature gradient of 0,5—3°C/h, is cooled to −2 to −6°C and then kept within this temperature range for at least 30 minutes, then

(iii)  the mother liquor present is run off at this temperature and

(iv)   the solid then present in the tube crystalliser is melted by heating.

2. Precoss according to claim 1 for preparing technically pure 2,4-diisocyanatotoluene, characterised in that

(iv)   the solid present in the tube crystalliser and obtained according to step (iii) is partly melted by heating, while maintaining a temperature gradient of 0,5—3°C/h, until the remaining solid has a content of 2,4-diisocyanatotoluene of at least 98% and then

(v)    the technically pure 2,4-diisocyanatotoluene thus obtained is isolated, after being melted, from the tube crystalliser.

3. Process according to claims 1 and 2, characterised in that an isomer mixture consisting essentially of 75 to 85% by weight of 2,4-diisocyanatotoluene and 15 to 25% by weight of 2,6-diisocyanatotoluene is used as the starting mixture.

## Revendications

1. Procédé de séparation de 2,4-diisocyanatotoluène pur ou de mélanges isomères présentant une teneur enrichie en 2,4-diisocyanatotoluène, à partir de mélanges isomères consistant essentiellement en 2,4- et en 2,6-diisocyanatotoluène ayant une teneur en 2,4-diisocyanatotoluène de 65—85% en poids et en 2,6-diisocyanatotoluène de 15—35% en poids, caractérisé en ce que le mélange isomère à employer comme matière première, dans un cristallisoir tubulaire consistant en un ou plusieurs tubes verticaux raccordés en parallèle sur lesquels ou entre lesquels une admission d'agent de refroidissement ou de chauffage uniforme et non inhibée est possible:

(i)    est refroidi à une température de +4°C à +9°C,

(ii)   puis le mélange est refroidi en respectant un gradient de température de 0,5—3°C/heure à −2 à −6°C, étant ensuite laissé pendant au moins 30 minutes dans cet intervalle de température,

(iii)  puis le liqueur-mère présente est déchargée à cette température et

(iv)   la substance solide, présente après cela dans le cristallisoir tubulaire, est amenée à l'état fondu par chauffage.

2. Procédé selon la revendication 1 pour la préparation de 2,4-diisocyanatotoluène techniquement pur, caractérisé en ce que

(iv)   on fond partiellement la substance solide obtenue selon le stade (iii) et qui est présente dans le cristallisoir tubulaire, tout en respectant un gradient de température de 0,5 à 3°C/heure, jusqu'à ce que la substance solide restante présente une teneur en 2,4-diisocyanatotoluène d'au moins 98%,

(v)    on isole ensuite le 2,4-diisocyanatotoluène techniquement pur ainsi obtenu après fusion à partir du cristallisoir tubulaire.

3. Procédé selon les revendications 1 et 2, caractérisé en ce qu'on utilise comme mélange initial un mélange isomère consistant essentiellement en 75 à 85% en poids de 2,4-diisocyanatotoluène et en 15 à 25% en poids de 2,6-diisocyanatotoluène.